(19) Europäisches Patentamt
European Patent Office
Office européen des brevets

(11) **EP 2 574 375 A1**

(12) **EUROPEAN PATENT APPLICATION**

(43) Date of publication:
**03.04.2013 Bulletin 2013/14**

(51) Int Cl.:
*A61N 7/02* (2006.01)  *A61B 19/00* (2006.01)

(21) Application number: **11182849.7**

(22) Date of filing: **27.09.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Koninklijke Philips Electronics N.V.
5621 BA Eindhoven (NL)**

(72) Inventors:
• **Mougenot, Charles
5600 AE Eindhoven (NL)**
• **Kohler, Max O. K.
5600 AE Eindhoven (NL)**

(74) Representative: **Damen, Daniel Martijn
Philips
Intellectual Property & Standards
P.O. Box 220
5600 AE Eindhoven (NL)**

(54) **Therapeutic apparatus for sonicating a moving target**

(57)  A therapeutic apparatus (300, 400, 500) comprising a high intensity focused ultrasound system (302) comprising an ultrasound transducer (306). The ultrasound transducer has an electronically adjustable focus (318). The high intensity focused ultrasound system has a beam deflection zone (322, 608, 704, 1010). The intensity of ultrasound at the electronically adjustable focus divided by the acoustic power emitted is above a predetermined threshold (606, 1008) within the beam deflection zone. The therapeutic apparatus further comprises a processor (328) for controlling the therapeutic apparatus. Execution of machine executable instructions (350, 352, 354) causes the processor to: receive (102, 202) real time medical data (342, 424, 506) descriptive of the location of a moving target (320, 802); adjust (104, 204) the electronically adjustable focus to target the moving target using the real time medical data; and sonicate (106, 206) the moving target when the moving target is within the beam deflection zone.

FIG. 1

start ~100

receive real time medical data ~102

adjust the electronically adjustable focus to target the moving target using the real time medical data ~104

sonicate the target when the moving target is within the beam deflection zone ~106

108
sonication finished? — No / Yes

end ~110

EP 2 574 375 A1

**Description**

TECHNICAL FIELD

The invention relates to high intensity focused ultrasound, in particular to the sonication of a moving target.

BACKGROUND OF THE INVENTION

Ultrasound is quickly becoming a desired approach for specific therapeutic interventions. In particular, the use of high intensity focused ultrasound is currently being used as an approach for thermal therapeutic intervention for uterine fibroids and has been examined for possible uses in the treatment of liver, brain, prostate, and other cancerous lesions. Ultrasound therapy for tissue ablation works by sonicating a tissue of interest with high intensity ultrasound that is absorbed and converted into heat, raising the temperature of the tissues. As the temperature rises coagulative necrosis of the tissues may occurs resulting in immediate cell death. The transducers used in therapy can be outside the body or be inserted into the body e.g. through blood vessels, urethra, rectum etc.

In high intensity focused ultrasound an array of transducer elements are used to form an ultrasonic transducer. Supplying alternating current electrical power to the transducer elements causes them to generate ultrasonic waves. The ultrasonic waves from each of the transducer elements either adds constructively or destructively. By controlling the phase of alternating current electrical power supplied to each of the transducer elements the focal point or volume into which the ultrasound power is focused may be controlled.

Mechanical displacement of the HIFU transducer allows large displacements of around 10cm which is very convenient to center the focal point on the volume to treat. But mechanical displacement is rather slow compared to the heating duration and the motion speed of the target.

SUMMARY OF THE INVENTION

The invention provides for a therapeutic apparatus, a method of operating a therapeutic apparatus and a computer program product in the independent claims. Embodiments are given in the dependent claims.

In many clinical situations there may be external and/or internal motion of the subject which is being sonicated. It is therefore beneficial to track the motion of a moving target when sonicating. Present solutions include gating the ultrasound and sonicating only when the target is in a particular location. It would be beneficial to be able to track the position of a target and adjust the focus of the ultrasound to decrease the time required to perform a sonication.

Mechanical steering of the focus may be too slow. Embodiments of the invention may provide a means of electronically steering the focus during sonication to reduce the total sonication time required for a moving target. Embodiments may enlarge the ablation and track the target tissue during the High Intensity Focused Ultrasound (HIFU) heating by using of electronic steering of the focal point. A deflection of the focal point can be performed by changing the phase on each individual channel of the phased array transducer to form a constructive interference at the wanted location. Electronic steering is very fast since there is no speed limitation except the minimal time to update electrical signal. Thus electronic steering allows changing the location of the focal point in typically less than a few milliseconds and it induces no perturbation on thermal maps. But electronic steering of the focal point induces intensity decrease as function of the amplitude of the deflection. As a consequence the electronic steering is limited to a small displacement within a beam deflection zone to avoid excessive intensity decreases especially since the remaining energy not deposited in the focus is absorbed in some uncontrolled location in the patient.

A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, punched tape, punch cards, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. References to a computer-readable storage medium should be interpreted as possibly being multiple computer-readable storage mediums. Various executable components of a program or programs may be stored in different locations. The computer-readable storage medium may for instance be multiple computer-readable storage medium within the same computer system. The computer-readable storage medium may also be computer-readable storage medium distributed amongst multiple computer systems or computing devices.

'Computer memory' or 'memory' is an example of a computer-readable storage medium. Computer memory is any memory which is directly accessible to a processor. Examples of computer memory include, but are not limited to: RAM memory, registers, and register files. References to 'computer memory' or 'memory' should be interpreted as possibly being multiple memories. The memory may for instance be multiple memories within the same computer system the memory may also be multiple memories distributed amongst multiple computer systems or computing devices.

'Computer storage' or 'storage' is an example of a computer-readable storage medium. Computer storage is any non-volatile computer-readable storage medium. Examples of computer storage include, but are not limited to: a hard disk drive, a USB thumb drive, a floppy drive, a smart card, a DVD, a CD-ROM, and a solid state hard drive. In some embodiments computer storage may also be computer memory or vice versa. References to 'computer storage' or 'storage' should be interpreted as possibly being multiple storage devices. The storage may for instance be multiple storage devices within the same computer system or computing device. The storage may also be multiple storages distributed amongst multiple computer systems or computing devices.

A 'processor' as used herein encompasses an electronic component which is able to execute a program or machine executable instruction. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. Many programs have their instructions performed by multiple processors that may be within the same computing device or which may even be distributed across multiple computing devices.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, tactile screen, pointing stick, graphics tablet, joystick, gamepad, webcam, headset, gear sticks, steering wheel, pedals, wired glove, dance pad, remote control, one or more buttons, one or more switches, and an accelerometer are all examples of user interface components which enable the receiving of information or data from an operator.

A 'hardware interface' as used herein encompasses an interface, which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

Magnetic Resonance (MR) data is defined herein as being the recorded measurements of radio frequency signals emitted by atomic spins by the antenna of a Magnetic resonance apparatus during a magnetic resonance imaging scan. A Magnetic Resonance Imaging (MRI) image is defined herein as being the reconstructed two or three dimensional visualization of anatomic data contained within the magnetic resonance imaging data. This visualization can be performed using a computer.

Thermographic data is data which is descriptive of the spatially dependent temperature within at least a portion of a subject. For example ultrasound data acquired by a diagnostic ultrasound system or magnetic resonance data may be or comprise thermographic data.

Magnetic Resonance thermometry data is defined herein as being the recorded measurements of radio frequency signals emitted by atomic spins by the antenna of a Magnetic resonance apparatus during a magnetic resonance imaging scan which contains information which may be used for magnetic resonance thermometry. Magnetic resonance thermometry data is an example of thermographic data. Magnetic resonance thermometry functions by measuring changes in temperature sensitive parameters. Examples of parameters that may be measured during magnetic resonance thermometry are: the proton resonance frequency shift, the diffusion coefficient, or changes in the T1 and/or T2 relaxation time may be used to measure the temperature using magnetic resonance. The proton resonance frequency shift is temperature dependent, because the magnetic field that individual protons, hydrogen atoms, experience depends upon the surrounding molecular structure. An increase in temperature decreases molecular screening due to the temperature affecting the hydrogen bonds. This leads to a temperature dependence of the proton resonant frequency.

The proton density depends linearly on the equilibrium magnetization. It is therefore possible to determine temperature changes using proton density weighted images.

The relaxation times T1, T2, and T2-star (sometimes written as T2*) are also temperature dependent. The reconstruction of T1, T2, and T2-star weighted images can therefore be used to construct thermal or temperature maps.

The temperature also affects the Brownian motion of molecules in an aqueous solution. Therefore pulse sequences which are able to measure diffusion coefficients such as a pulsed diffusion gradient spin echo may be used to measure temperature.

One of the most useful methods of measuring temperature using magnetic resonance is by measuring the proton resonance frequency (PRF) shift of water protons. The resonant frequency of the protons is temperature dependent. As the temperature changes in a voxel the frequency shift will cause the measured phase of the water protons to change. The temperature change between two phase images can therefore be determined. This method of determining temperature has the advantage that it is relatively fast in comparison to the other methods. The PRF method is discussed in greater detail than other methods herein. However, the methods and techniques discussed herein are also applicable to the other methods of performing thermometry with magnetic resonance imaging.

An 'ultrasound window' as used herein encompasses a window which is able to transmit ultrasonic waves or energy. Typically a thin film or membrane is used as an ultrasound window. The ultrasound window may for example be made of a thin membrane of BoPET (Biaxially-oriented polyethylene terephthalate).

In one aspect the invention provides for a therapeutic apparatus comprising a high-intensity focused ultrasound system comprising an ultrasound transducer. The ultrasound transducer has an electronically adjustable focus. The ultrasound transducer may comprise multiple transducer elements. The amplitude and in particular the phase of alternating current electrical power provided to the individual elements making up the ultrasound transducer enables the focus to be electronically adjusted. The ultrasound generated by each element adds either constructively or destructively with the ultrasound from other elements. By controlling the phase this may be used to deflect or adjust the location of the focus. The high-intensity focused ultrasound system has a beam deflection zone. The ultrasound transducer is configured for generating acoustic power when supplied with alternating electrical current power. When the ultrasound transducer comprises multiple transducer elements these elements may be configured for generating acoustic power with a controllable phase. The intensity of the ultrasound at the electronically adjustable focus divided by the acoustic power emitted is above a predetermined threshold within the beam deflection zone. In other words the beam deflection zone is a zone where the intensity at the focus divided by the acoustic power is above the predetermined threshold. As the beam is deflected electronically the intensity at the focus divided by the acoustic power may decrease. If the electronically adjustable focus is diverted from the natural focus too far then the intensity at the focus divided by the acoustic power will be greatly reduced.

A beam deflection zone essentially defines a region where the ultrasound at the location of electronically adjustable focus is sufficiently powerful to be useful for sonicating a target. The therapeutic apparatus further comprises a memory for storing machine executable instructions. The therapeutic apparatus further comprises a processor configured for controlling the therapeutic apparatus. Execution of the machine executable instructions causes the processor to receive real time medical data. The real time medical data is descriptive of the location of a moving target. By real time it is meant that the location of the moving target is accurate within a predetermined time. That is to say that the real time medical data is useful for identifying a current location of the moving target. The real time medical data is used to target the moving target. If the moving target is in a different location or in a location greater than a predetermined distance then the real time medical data is not useful for identifying the location of the moving target. Execution of the machine executable instructions further causes the processor to adjust the electronically adjustable focus to target the moving target using the real time medical data. Execution of the machine executable instructions causes the processor to sonicate the moving target when the moving target is within the beam deflection zone. In some embodiments the moving target is continually sonicated when the moving target is within the beam deflection zone. This embodiment may be beneficial because the moving target is sonicated for a longer duration than if the sonication is only gated when the moving target is in a particular location. This may have the advantage of being able to perform the sonication more rapidly.

In another embodiment the moving target is located at least partially using real time tracking of the target within the real time medical data. The real time medical data may describe the location of the target with a predetermined delay. The real time tracking may use a model or predict the trajectory of the target based on previous trajectory or behavior of the target. This embodiment may be beneficial because it allows more accurate determination of the location of the moving target and therefore more accurate targeting during sonication.

In another embodiment execution of the instructions causes the processor to receive a motion tracking model. The motion tracking model is configured for predicting the location of the moving target using the real time medical data. In some embodiments, the motion tracking model is adapted or modified based on the motion of the target zone. As the moving target is tracked the motion tracking model could be adapted or modifed using the real time medical data.

The electronically adjustable focus is adjusted using the predicted location of the moving target. Essentially the real time medical data is used as an input to the motion tracking model which outputs a predicted location. The electronically adjustable focus is then adjusted to the predicted location. Many types of motion within a subject such as respiration are periodic or repeat over different timescales. The motion of an internal anatomy can be therefore predicted by measuring various anatomical parameters. For example the position of the diaphragm is useful in predicting the location of various organs within the thoracic cavity. The beating of a subject's heart may also cause the dislocation of certain anatomical features. Models for predicting the location of internal anatomical features are known in the art. Such a known model could be used and also adapted to the individual patient. The adaptation could be done using measurements that capture the motion of the organs of interest before starting therapy.

In another embodiment the moving target comprises at least one localized volume. A localized volume as used herein is a volume of a predetermined size. For instance the high-intensity focused ultrasound system may be used to target a single point within a subject. However, the high-intensity focused ultrasound system does produce sonication within a finite difference. In some embodiments the localized volume is the smallest volume that the high-intensity focused ultrasound system may sonicate.

In another embodiment the moving target comprises multiple localized volumes. The electronically adjustable focus is targeted to the moving target by specifying a sequence of localized volumes chosen from the multiple localized volumes. Execution of the instructions further causes the processor to determine the sequence in accordance with the real time medical data. In other words the moving target may comprise multiple locations to be sonicated. Not all of these multiple localized volumes may be within the beam deflection zone. Therefore the real time medical data may be used to determine which of the multiple localized volumes should be sonicated. This embodiment may be advantageous because it may allow the more rapid sonication of the moving target because the localized volumes to be sonicated can be determined in real time.

In another embodiment Execution of the instructions further causes the processor to receive an ultrasonic calibration. The ultrasonic calibration is descriptive of the spatially dependent intensity of the ultrasound at the electronically adjustable focus within the beam deflection zone. In other words the ultrasonic calibration contains data which is descriptive of how strong or intense the focused ultrasound is within different locations of the beam deflection zone. Execution of the instructions further causes the processor to determine a sonication duration for each localized volume in accordance with the ultrasonic calibration and the real time medical data. The real time medical data and the ultrasonic calibration can be used to determine how long a particular localized volume should be sonicated. This is important because some sonication points may be within the deflection zone for a shorter period of time and some points may receive more ultrasonic energy due to the location within the beam deflection zone. Determining a sonication duration for each localized volume is therefore beneficial to perform a better control of the temperature rise and/or thermal dose delivery of the moving target. This control can be set to reach either a temperature rise and/or thermal dose delivery uniform over the target or any predefine delivery distribution over the target such as a distribution reducing the total energy to emit. In another embodiment the moving target comprises at least one path. As used herein a path is a continuous volume which may be sonicated by moving the electronically adjustable focus in one or more directions.

In another embodiment the electronically adjustable focus follows a trajectory along the at least one path. Execution of the instructions further causes the processor to determine the trajectory in accordance with the real time medical data. In this embodiment the electronically adjustable focus is adjusted to sonicate along a trajectory, however the portion of the trajectory which is sonicated needs to be within the beam deflection zone. This may be beneficial because the trajectory may be more rapidly sonicated.

In another embodiment execution of the instructions further causes the processor to receive an ultrasonic calibration. The ultrasonic calibration is descriptive of the spatially dependent intensity of the ultrasound at the electronically adjustable focus with the beam deflection zone. Each of the at least one path is divided into portions. Execution of the instructions further causes the processor to determine a sonication duration in accordance with the ultrasonic calibration and the real time medical data for each of the portions. The therapeutic apparatus is configured to sonicate each of the portions for the sonication duration. The benefits of this embodiment are equivalent to many of the benefits for the embodiment where a sonication duration is determined for one or more localized volumes.

In another embodiment the location of the moving target is predicted using dynamical analysis of the real time medical imaging data. In dynamic analysis the motion may be deduced from an optical flow method. Segmentation of the image may be performed based on the detection of contours. Optical flow is based on cross-correlation between two images from a dataset to identify matching locations of each voxel. This method has the advantage that it is relatively fast and gives displacement of each voxel rather than displacement of tissue contour.

In another embodiment the real time medical data is real time medical image data. Execution of the instructions further causes the processor to acquire the real time medical image data using the medical imaging system.

In another embodiment the medical imaging system is a magnetic resonance imaging system.

In another embodiment the medical imaging system is a diagnostic ultrasound imaging system. The diagnostic ultrasound imaging system may have an additional transducer for making diagnostic ultrasound measurements. In yet other embodiments the transducer for the diagnostic ultrasound system uses either a diagnostic ultrasound transducer built into the ultrasound transducer of the high-intensity focused ultrasound system.

In another embodiment the medical image data comprises motion data and real time thermographic data. The moving target is located using the motion data.

In another embodiment the sonication of the moving target is controlled in accordance with the thermographic data. For instance the thermographic data may be magnetic resonance thermometry data. Ultrasound imaging techniques may also be used to acquire temperature data. The acquisition of real time thermographic data enables the calculation of a thermal dose delivered to a region of the subject. Regions that have been under-sonicated may be sonicated more or given preference for sonication and regions which have been fully sonicated can be left out for further sonication. This has the advantage of making the sonication more efficient.

In another aspect the invention provides for a method of operating a therapeutic apparatus. The therapeutic apparatus comprises a high-intensity focused ultrasound system comprising an ultrasound transducer. The ultrasound transducer has an electronically adjustable focus. The high-intensity focused ultrasound system has a beam deflection zone. The ultrasound transducer is configured for generating acoustic power when supplied with alternating current electrical power. The intensity of ultrasound at the electronically adjustable focus divided by the acoustic power emitted is above a predetermined threshold within the beam deflection zone. The method comprises the step of receiving real time medical data. The real time medical data is descriptive of the location of the moving target. The method further comprises the step of adjusting the electronically adjustable focus to target the moving target using the real time medical data. The method further comprises the step of sonicating the moving target when the moving target is within the beam deflection zone. The benefits of this method have been previously discussed.

In another aspect the invention provides for a computer program product comprising machine executable instructions for operating a therapeutic apparatus. The therapeutic apparatus comprises a high-intensity focused ultrasound system comprising an ultrasound transducer. The ultrasound transducer has an electronically adjustable focus. The high-intensity focused ultrasound system has a beam deflection zone. The ultrasound transducer is configured for generating acoustic power when supplied with alternating current electrical power. The intensity of ultrasound at the electronically adjustable focus divided by the acoustic power emitted is above a predetermined threshold within the beam deflection zone. The therapeutic apparatus further comprises a processor configured for controlling the therapeutic apparatus. Execution of the machine executable instructions causes the processor to receive real time medical data. Execution of the machine executable instructions further causes the processor to adjust the electronically adjustable focus to target the moving target using the real time medical data. Execution of the machine executable instructions further causes the processor to sonicate the moving target when the moving target is within the beam deflection zone. The benefits of this computer program product have been previously discussed.

BRIEF DESCRIPTION OF THE DRAWINGS

In the following preferred embodiments of the invention will be described, by way of example only, and with reference to the drawings in which:

Fig. 1 shows a flow chart which illustrates a method according to an embodiment of the invention;
Fig. 2 shows a flow chart which illustrates a method according to a further embodiment of the invention;
Fig. 3 illustrates a therapeutic apparatus according to an embodiment of the invention;
Fig. 4 illustrates a therapeutic apparatus according to a further embodiment of the invention;
Fig. 5 illustrates a therapeutic apparatus according to a further embodiment of the invention;
Fig. 6 shows a plot of the normalized intensity of ultrasound generated by a high-intensity focused ultrasound sensor as a function of deflection perpendicular to the beam axis;
Fig. 7 shows a plot of the normalized intensity of ultrasound generated by a high-intensity focused ultrasound sensor as a function of deflection along the beam axis;
Figs. 8A, 8B, 8C, 8D, and 8E illustrate a subject with a moving target;
Figs. 9A, 9B, 9C, 9D, and 9E show a top view of the motion shown in Figs. A8 through 8E;
Fig. 10 illustrates a planar representation of linear periodic motion;
Fig. 11 illustrates the sonication time for the periodic motion shown in Fig. 10;
Fig. 12 illustrates a planar representation of a curved periodic motion trajectory;
Fig. 13 illustrates the sonication time for the periodic motion shown in Fig. 12;
Fig. 14 illustrates a planar representation of linear periodic motion with five sonication points;
Fig. 15 illustrates the sonication time for the periodic motion shown in Fig. 14;
Fig. 16A, 16B, 16C, 16D, and 16E illustrate a subject with a moving target;
Fig. 17 is analogous to figure 14, except that there is higher energy deposition in each point;
Fig. 18 illustrates the sonication time for the periodic motion shown in Fig. 17;
Fig. 19 shows the number of point to sonicated during each part time part of the cycle for the example shown in Figs. 17 and 18;

Fig. 20 is analogous to figure 17, except that multiple points are sonicated simultaneously;
Fig. 21 illustrates the sonication time for the periodic motion shown in Fig. 20;
Fig. 22 is analogous to Fig. 20, except that the sonication paths are curved;
Fig. 23 illustrates the sonication time for the periodic motion shown in Fig. 23;
Fig. 24A, 24B, 24C, 24D, and 24E illustrate a subject with a moving target;
Fig. 25A, 25B, 25C, 25D, and 25E illustrate a subject with a moving target;
Fig. 26A, 26B, 26C, 26D, and 26E illustrate a subject with a moving target; and
Fig. 27 shows an example of sonications for continuous linear displacement;

## DETAILED DESCRIPTION OF THE EMBODIMENTS

Like numbered elements in these figures are either equivalent elements or perform the same function. Elements which have been discussed previously will not necessarily be discussed in later figures if the function is equivalent.

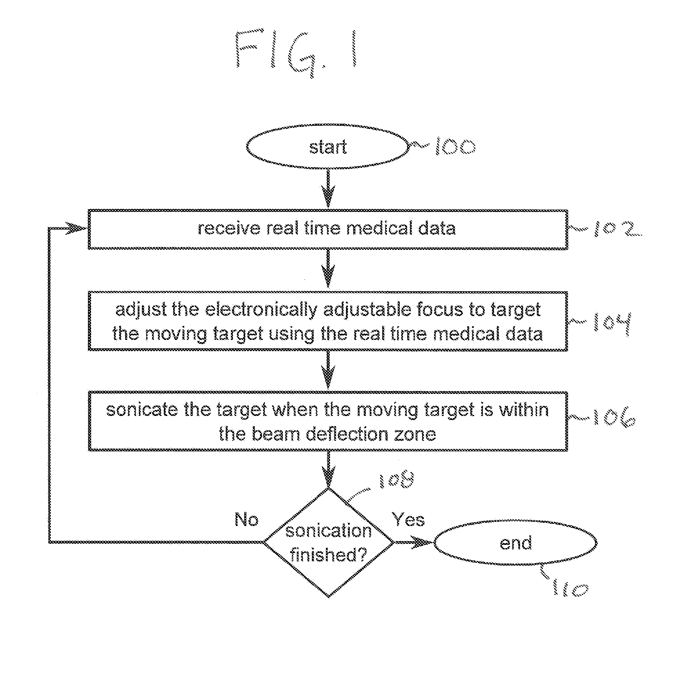

Fig. 1 shows a flow diagram which illustrates a method according to an embodiment of the invention. In step 100 the method starts. Next in step 102 real medical data is received. Next in step 104 the electronically adjustable focus is adjusted to target the moving target using the real time medical data. Next in step 106 the target is sonicated when the moving target is within the beam deflection zone. This may also include sonicating a portion of the target which is within the beam deflection zone. Box 108 is a decision box. If the sonication is not finished then the method returns to step 102. Steps 102-106 are repeated in a continuous loop until the sonication is finished. In this way real time medical data is received and used to continually adjust the sonication. When the sonication is finished then the method ends in step 110.

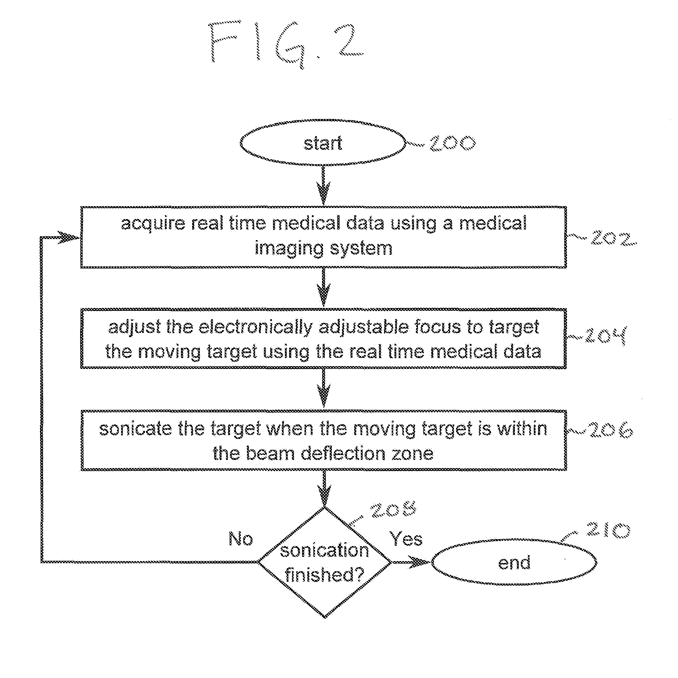

Fig. 2 shows a flow diagram which illustrates a further method according to an embodiment of the invention. The method shown in Fig. 2 starts at block 200. Next in step 202 real time medical image data is acquired using a medical imaging system. In this case the acquisition of real time medical data by a medical imaging system is equivalent to receiving the real time medical data. Next in step 204 the electronically adjustable focus is adjusted to target the moving target using the real time medical data. In step 206 the target is sonicated when the moving target is within the beam deflection zone. Again portions of the target which are within the beam deflection zone may be sonicated also. Block 208 is a decision block. If the sonication is not finished then steps 202, 204, and 206 are repeated in a loop until the sonication is finished. Next in step 210 the method ends when the sonication is finished.

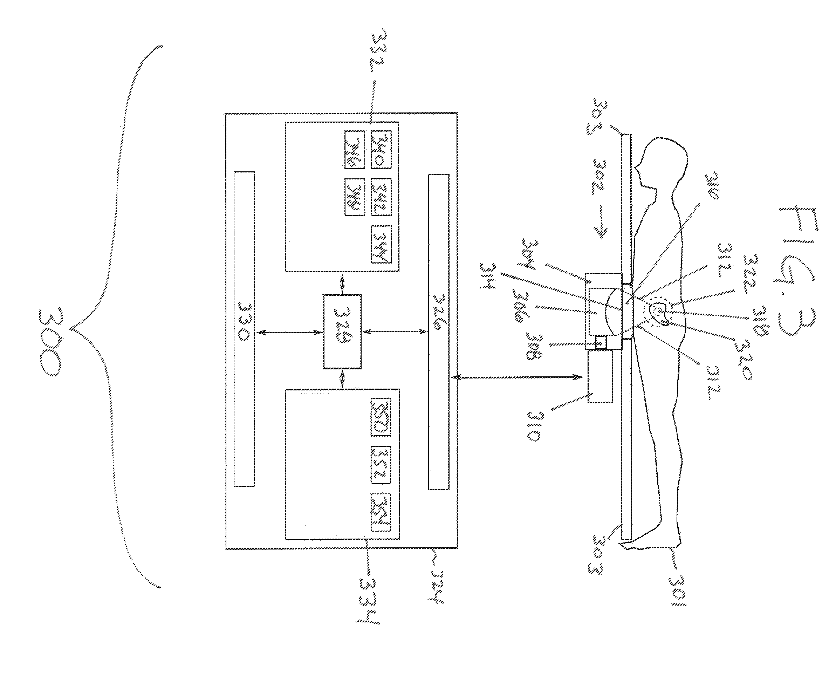

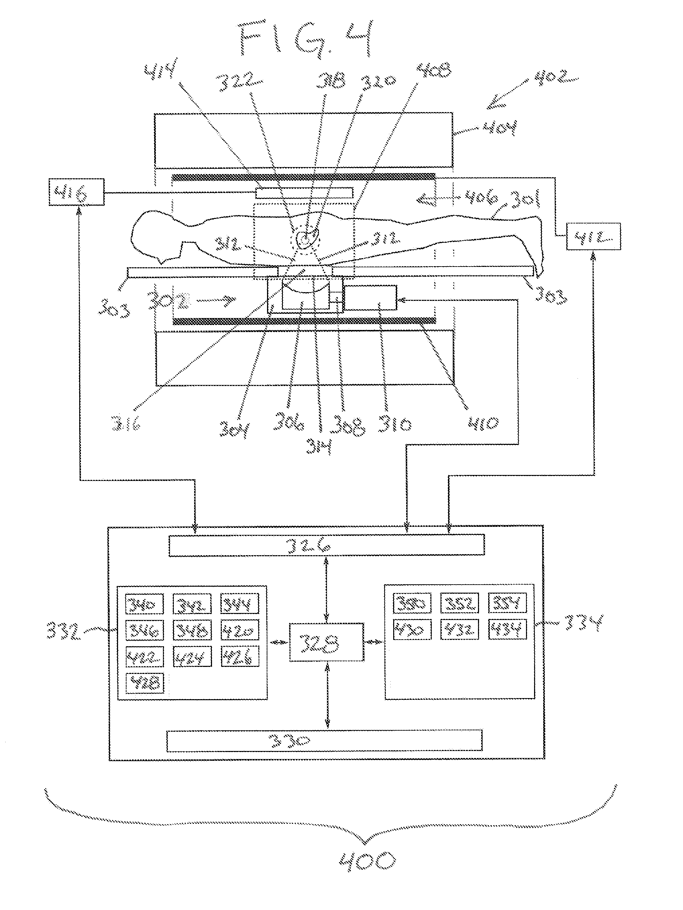

Fig. 3 illustrates a therapeutic apparatus 300 according to an embodiment of the invention. In addition to the components, the embodiment shown in Fig. 4 comprises a temperature treatment system which is a high-intensity focused ultrasound system 302 for sonicating a subject 301. The high-intensity focused ultrasound system is mounted below a subject support 303. The subject 301 is resting on the subject support 303. The high-intensity focused ultrasound system comprises a fluid-filled chamber 304. Within the fluid-filled chamber 304 is an ultrasound transducer 306. Although it is not shown in this figure the ultrasound transducer 306 may comprise multiple ultrasound transducer elements each capable of generating an individual beam of ultrasound. This may be used to steer the location of a sonication point 318 electronically by controlling the phase and/or amplitude of alternating electrical current supplied to each of the ultrasound transducer elements.

The ultrasound transducer 306 is connected to a mechanism 308 which allows the ultrasound transducer 306 to be repositioned mechanically. The mechanism 308 is connected to a mechanical actuator 310 which is adapted for actuating the mechanism 308. The mechanical actuator 310 also represents a power supply for supplying electrical power to the ultrasound transducer 306. In some embodiments the power supply may control the phase and/or amplitude of electrical power to individual ultrasound transducer elements. The ultrasound transducer 306 generates ultrasound which is shown as following the path 312. The ultrasound 312 goes through the fluid-filled chamber 308 and through an ultrasound window 314. In this embodiment the ultrasound then passes through a gel pad 316. The gel pad is not necessarily present in all embodiments but in this embodiment there is a recess in the subject support 303 for receiving a gel pad 316. The gel pad 316 helps couple ultrasonic power between the transducer 306 and the subject 301. After passing through the gel pad 316 the ultrasound 312 passes through the subject 301 and is focused to a sonication point 318. The sonication point is understood to be a finite volume or localized volume to which the ultrasound is focused. The sonication point 318 is being focused within a moving target 320. The sonication point 318 may be moved through a combination of mechanically positioning the ultrasonic transducer 306 and electronically steering the position of the sonication point 318.

The region marked 322 is the beam deflection zone. The beam deflection zone is a region where the electronically adjustable focus 318 can be adjusted such that the intensity of ultrasound at the electronically adjustable focus 318 divided by the acoustic power emitted by the transducer 306 is above a predetermined threshold. In this example the entire moving target 320 is within the beam deflection zone 322. However, in other embodiments or instances only a portion of the moving target 320 may be within the beam deflection zone 322. Also for some time periods the moving target may be entirely or partially within the beam deflection zone 322 and other time instances it may be partially or completely out of the beam deflection zone 322.

The high intensity focused ultrasound system 302 is shown as being connected to a hardware interface 326 of the computer 324. The hardware interface 326 is connected to a processor 328. The hardware interface 326 enables the processor 328 to send and receive data and commands to control the operation and function of the therapeutic apparatus 300. The processor 328 is further connected to a user interface 330, computer storage 332 and computer memory 334.

The computer storage 332 is shown as containing a treatment plan 340. The treatment plan 340 may contain detailed instructions or controls for sonicating or specifying the sonication of the moving target 320. The computer storage 332 is further shown as containing real time medical data 342. The real time medical data 342 may be received from some other instrument or device which is capable of generating the real time medical data. For instance the therapeutic apparatus 300 may be connected or networked to a medical imaging system. The computer storage 332 is further shown as containing sonication control commands 344 that were generated using the real time medical data 342. The computer storage 332 is further shown as containing an ultrasonic calibration 346. The ultrasonic calibration is descriptive of the spatially dependent intensity of ultrasound divided by the acoustic power emitted. Further shown in the computer storage 332 is a set of sonication durations 348. The sonication durations 348 are for different portions or parts of the moving target 320. They may have been calculated using the ultrasonic calibration 346.

The computer memory 334 is shown as containing a control module 350. The control module 350 contains computer executable code which enables the processor 328 to control the operation and function of the therapeutic apparatus 300. The computer memory 334 is further shown as containing a sonication control command generation module 352. The sonication control command generation module 352 contains computer executable code which enables the processor 328 to generate the sonication control commands 344 using the real time medical data 342. The computer memory 334 is further shown as containing a motion tracking module 354. This is an optional module in some embodiments where the sonication command generation module 352 uses the motion tracking module 354 and the real time medical data 342 to predict the current location of the moving target 320. This enables more accurate sonication of the moving target 320.

Fig. 4 shows a therapeutic apparatus 400 according to a further embodiment of the invention. The therapeutic apparatus 400 shown in figure 4 is similar to the therapeutic apparatus 300 shown in Fig. 3. The therapeutic apparatus 400 comprises a magnetic resonance imaging system 402. The magnetic resonance imaging system comprises a magnet 404. The magnet 404 is a cylindrical type superconducting magnet with a bore 406 through the center of it. The magnet has a liquid helium cooled cryostat with superconducting coils. It is also possible to use permanent or resistive magnets. The use of different types of magnets is also possible for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 406 of the cylindrical magnet there is an imaging zone 408 where the magnetic field is strong and uniform enough to perform magnetic resonance imaging.

Within the bore 406 of the magnet there is also a set of magnetic field gradient coils 410 which are used for acquisition of magnetic resonance data to spatially encode magnetic spins within the imaging zone 408 of the magnet 404. The magnetic field gradient coils are connected to a magnetic field gradient coil power supply 412. The magnetic field gradient coils 410 are intended to be representative. Typically magnetic field gradient coils contain three separate sets of coils for spatially encoding in three orthogonal spatial directions. A magnetic field gradient power supply 412 supplies current to the magnetic field gradient coils 410. The current supplied to the magnetic field coils is controlled as a function of time and may be ramped or pulsed.

Adjacent to the imaging zone 408 is a radio-frequency coil 414 for manipulating the orientations of magnetic spins within the imaging zone 408 and for receiving radio transmissions from spins also within the imaging zone. The radio-frequency coil may contain multiple coil elements. The radio-frequency coil may also be referred to as a channel or an antenna. The radio-frequency coil 414 is connected to a radio frequency transceiver 416. The radio-frequency coil 414 and radio frequency transceiver 416 may be replaced by separate transmit and receive coils and a separate transmitter and receiver. It is understood that the radio-frequency coil 414 and the radio-frequency transceiver 416 are representative. The radio-frequency coil 414 is intended to also represent a dedicated transmit antenna and a dedicated receive antenna. Likewise the transceiver 416 may also represent a separate transmitter and receivers.

The computer storage 332 is shown as additionally containing a pulse sequence 420. A pulse sequence as used herein encompasses a set of instructions which enables the processor 328 to control the magnetic resonance imaging system 402 to acquire the magnetic resonance data 424. The computer storage 332 is further shown as containing magnetic resonance data 424 that was acquired using the magnetic resonance imaging system 402. The magnetic resonance data 424 is the real time medical data in this embodiment. The computer storage 332 is further shown as containing a magnetic resonance thermometry pulse sequence 422. The magnetic resonance thermometry pulse sequence 422 is a pulse sequence which enables the magnetic resonance imaging system 402 to acquire magnetic resonance thermometry data which comprises magnetic resonance thermometry data. The computer storage 332 is further shown as containing a magnetic resonance image 426 that has been reconstructed from the magnetic resonance data 424. The computer storage 332 is further shown as containing a temperature map 428 that has been reconstructed from the magnetic resonance data 424. In this case the magnetic resonance data comprises magnetic resonance thermometry data. The computer memory 334 is further shown as containing an image reconstruction module 430 which was used for reconstructing the magnetic resonance data 424 into the magnetic resonance image 426. The computer storage 334 is further shown as containing a temperature mapping module 432 which was used to reconstruct the temperature map 428 from the magnetic resonance data 424. In some embodiments the sonication control command generation module 352 uses the magnetic resonance image 426 and/or the temperature map 428 in generating the sonication control commands 344. For instance the magnetic resonance image may be the real time medical data and may provide an identification of the location of the moving target 320 within the subject. As another example the temperature map 428 may be used for identifying how long a particular region of the moving target 320 has been sonicated. This could be used for adjusting the sonication durations 348. In some embodiments the computer memory 334 contains an image segmentation module 434. The sonication control command generation module 352 may use the image segmentation module 434 with the magnetic resonance image 426 to identify the location of the moving target 320.

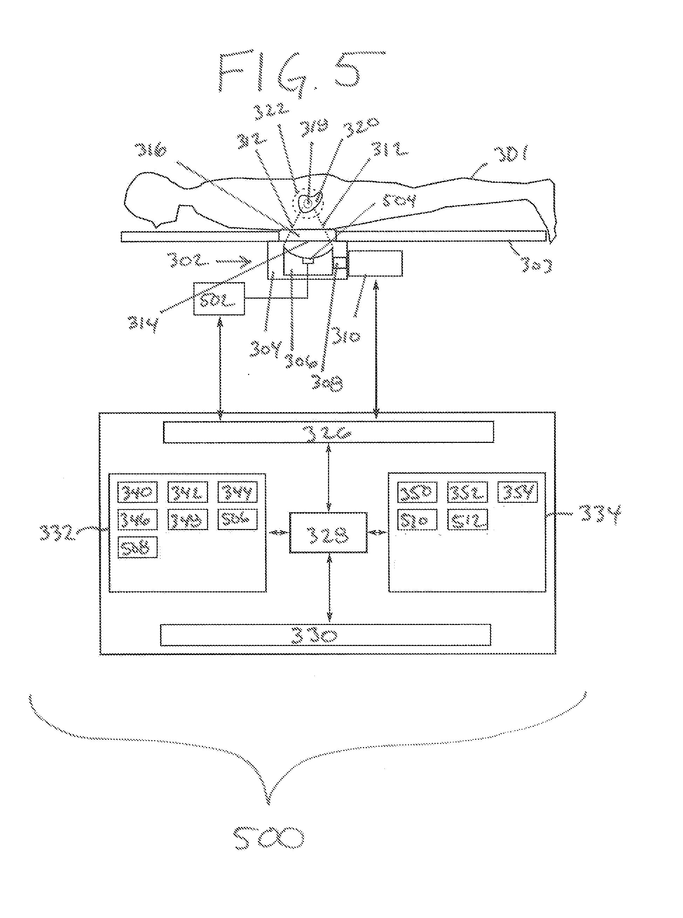

Fig. 5 shows a therapeutic apparatus 500 according to a further embodiment of the invention. The embodiment shown in Fig. 5 is similar to the embodiment shown in Fig. 3. In Fig. 5 a diagnostic ultrasound system 502 has been added to the therapeutic apparatus 500. A diagnostic ultrasound transducer 504 is incorporated into the ultrasound transducer 306. In some alternative embodiments the diagnostic ultrasound transmission 504 is separate from the ultrasound transducer 306. The diagnostic ultrasound transducer 504 is connected to the diagnostic ultrasound system 502. The diagnostic ultrasound system 502 is also connected to the hardware interface 326. The control module 350 in this embodiment is also adapted for controlling the diagnostic ultrasound system 502.

The computer storage 332 is shown as containing diagnostic ultrasound data 506. The diagnostic ultrasound data 506 in this embodiment is equivalent to real time medical data. The computer storage 332 is further shown as containing a diagnostic ultrasound image 508. The diagnostic ultrasound image 508 was reconstructed from the diagnostic ultrasound data 506. The computer memory 334 is further shown as containing an image reconstruction module 510. The image reconstruction module 510 contains computer executable code for reconstructing the diagnostic ultrasound image 508 from the diagnostic ultrasound data 506. The computer memory 334 is further shown as containing image segmentation module 512. The image segmentation module 512 contains computer executable code for identifying the location of the moving target 320 within the diagnostic ultrasound image 508. The sonication control command generation module 352 may use the image segmentation module 512 to identify the location of the moving target 320 when it is generating the sonication control commands 344.

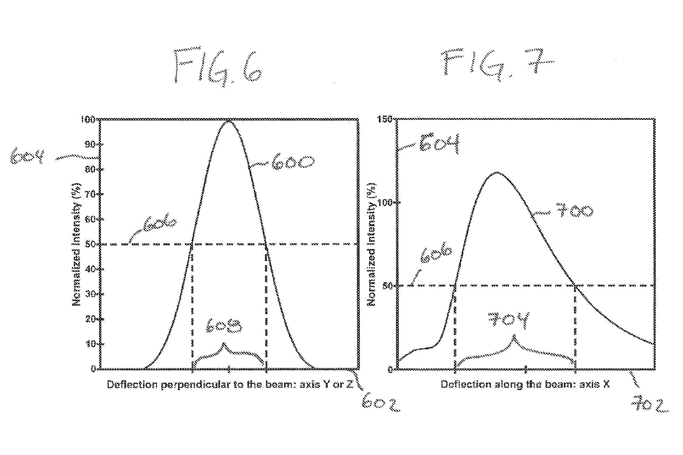

Fig. 6 shows a plot of the normalized intensity of ultrasound generated by a high-intensity focused ultrasound transducer. The plot on the x-axis is the deflection perpendicular to the beam, which corresponds to the y or z-axis of the transducer 602. The y-axis is the normalized intensity 604. The normalized intensity is the intensity of ultrasound at the electronically adjustable focus divided by the acoustic power emitted by the ultrasound transducer normalized by the intensity divided by the acoustic power at the geometrical focus of the typical concave HIFU transducer. For non concave HIFU transducer any other reference point such as the one generating the maximal intensity divided by acoustic power can be used. The dashed line 606 shows the normalized intensity at 50%. This may be one example of a predetermined threshold for which the normalized intensity is above. The region 608 may be an example of a beam deflection zone defined by the threshold 606.

Fig. 7 shows the normalized intensity 700 for deflection along the beam axis or the x-axis. The x-axis is the beam deflection along the transducer's x-axis. The y-axis is again the normalized intensity 604. The dashed line 606 shows the 50% normalized intensity. This is used to define the beam deflection zone 704 in Fig. 7.

Mainly three strategies exist for the treatment of mobile organs by MR-HIFU. The simple one consists to sonicate continuously independently of the motion. In that case, the heating spreads along the uncontrolled trajectory of motion of the target tissue. Alternatively, the sonication can be gated with the respiratory or the cardiac cycle to achieve heating only at the target location, but the heating duty cycle is usually too low to treat perfused organs such as liver or kidney. Alternatively the focal point can be moved electronically to compensate the target tissue displacement but the large focal point deflection required reduces the heating efficiency and restricts the use of simultaneous volumetric heating also based electronic steering.

Embodiments of the invention may use the tissue displacement to minimize the required electronic steering to achieve a more efficient heating in combination with volumetric heating. For example if heating of a volumetric circle is to be achieved on an organ moving along one direction, only displacement perpendicular to the motion direction may be necessary to sonicate this circle.

Figs. 6 and 7 illustrate the intensity drop while using deflection perpendicular to the beam axis (usually Foot-Head (FH) and -Left-Right (LR) direction) and along the beam axis (usually Anterior-Posterior (AP) direction). The reference to normalized the intensity is the intensity while focal point is located at the natural focus location defined by the geometric center of the typically concave transducer (all channels of the transducer in phase). However we can notice in particular case that the steering of the focal point along the beam axis toward the transducer can induce intensity increase (sometime up to 20%) because there is more energy at the proximity of the transducer.

Figs. 6 and 7 show the maximal deflection (Fig. 6) perpendicular to the beam axis and (Fig. 7) along the beam axis is typically defined by the threshold 50% of the intensity at the natural focus location. In this example, the threshold to define the maximal acceptable steering is usually taken to be 50% of the normalized intensity.

This maximal steering range is typically twice longer along the beam axis compared to steering range perpendicular to the beam axis. If a steering is used to move the focal point along the beam axis, thus the maximum steering range perpendicular to the beam axis would be smaller. The Fig. 6 and 7 display the intensity drop along two axes, but in practice this intensity drop is evaluated in 3D to process intensity compensation for all location by applying higher electrical power level on the transducer.

The treatment of mobile organ such as kidney or liver is problematic since the target tissue moves relative to the transducer which is usually in a fixed position attached to the therapeutic platform. If the motion is not taken into account, the heating induced by the static focal point is spread along the motion trajectory of the tissue which makes the heating less efficient and the healthy tissue may also be ablated. The first strategy proposed to overcome this issue was the use of sonications gated on the cardiac or respiratory periodic cycle. In that case the location of the heating is well localized but the heating remains relatively inefficient because the tissue is heated during only a small fraction of the motion cycle. To overcome this low duty cycle, the alternative solution consists to perform electronic steering to heat continuously the same part of the target tissue by applying deflection equal to the motion.

However the amplitude of the motion can be as large as or even larger than the maximal electronic steering range. For example, the upper part of the liver may move with an amplitude of 4 cm along Foot to Head (FH) direction which is larger than the typical steering range of HIFU systems of approximately 2 cm along this direction. In addition if the full steering range capability is used to perform compensation of tissue motion, high intensity power compensation factor has to be used to achieve the target intensity, which leaves it impossible to perform additional steering for enlarging the heated volume via volumetric sonication.

Embodiments of the invention may use the tissue or target motion to enlarge the energy delivery to a control extend with a smart combination of motion and focal point deflection. To obtain the planned energy delivery ablation an optimal use of the energy may be performed by minimizing the amplitude of the deflection by identifying the optimal part of the motion trajectory to perform the sonications for each target points.

The sonication parameters can include as function of time, the position of focal point (i.e., sonication trajectories), the shape of focal point (multiple or simple focal points or any pressure distribution), the acoustic power at the focus or/and the electrical power applied on the transducer (variable power level or use of duty cycle).

Embodiments of the invention may include a ultrasound transducer including multiple channels in order to control the location of the ultrasound energy delivery could be used for this purpose. The electronic displacement of the focal point can be characterized herein as a trajectory named "sonicated trajectory."

Embodiments of the invention may include a means to quantify motion. This may be any imaging modality which allows quantification of the motion such as Magnetic Resonance Imaging (MRI) or ultrasound imaging device could be used to characterize the motion. This characterization of the motion could be also obtained by a combination of several imaging modalities such as MRI (for the good spatial tissue contrast) and ultrasound (for the high temporal resolution). The global displacement of the whole target or the displacement of each points of the target volume can be used to characterize the motion using either rigid or elastic model. The trajectories considered could be evaluated in 1, 2 or 3 dimensions for periodic motion such as respiratory or cardiac cycle or a-periodic motion such as muscle contraction. A model including also combination of several type of motion could be also considered such as cardiac cycle and periodic cycle. External sensors can also be used to identify the location within the breathing or cardiac cycle such as respiratory bellows, cardiac ECG or VCG.

The displacement of the target point due to the motion of the tissue can be characterized by trajectory named "motion trajectory" for this document. The use of "sonicated trajectory" and "motion trajectory" allows to differentiate the type of target point displacement.

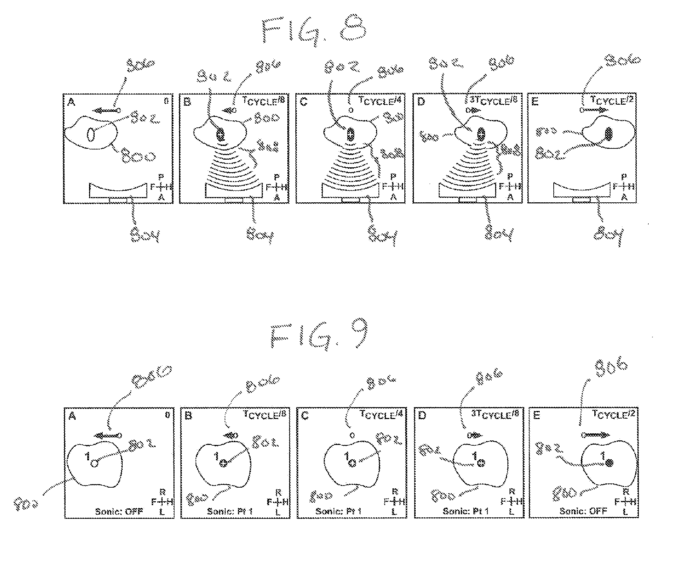

Fig. 8a, b, c, d and e show a subject 800 with a moving target 802. Each of the frames a, b, c, d and e are different time periods. The transducer is shown as item 804. The arrows or points labeled 806 shows the displacement of the moving target 802. When the moving target 802 is within the beam deflection zone it is sonicated with the ultrasound 808. The sonication is only performed in Figs. 8b, c and d. Fig. 8 shows the Side view of sonication of a target point moving along FH direction using motion tracking during part of the displacement. The arrow indicates for each time frame the tissue displacement relative to the central position.

Fig. 9 shows a top view of the same sonication that was shown in Fig. 8. Fig. 9 shows the top view of the same sonications as in Fig. 8 of a target point moving along FH direction using motion tracking during part of the displacement. The arrow indicates for each time frame the tissue displacement relative to the central position.

Single point heating:

To start with a simple case, it's possible to consider the heating of a single target tissue point (named 1) with a periodic linear motion trajectory crossing the geometric center of transducer. As illustrated in Fig. 8 and 9, the target point can be sonicated only during a part of the cycle to achieve the require ablation while restricting the amplitude of deflection used.

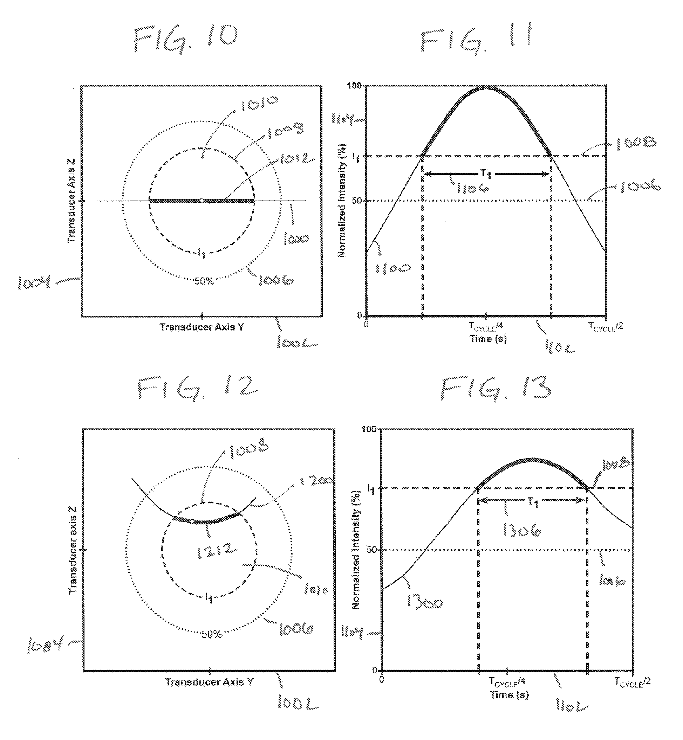

A temperature and/or dose control algorithm can process the amount of energy $E_1$ to deliver on this point for a half cycle $T_{CYCLE}/2$. All this energy can't be deliver in a single brief pulse because high power level can induce unwanted cavitation effect or because of hardware limitation such as transducer or amplifier over-heating. Based on the knowledge of the maximal acoustic power level $P_{MAX}$ apply at the focal point (either selected by user or based on system specification), the minimal required heating duration can be defined by $T^0_1 = E_1/P_{MAX}$. In some cases the heating duration may need to be split into several discrete times. For example, a target point could move in and out of the beam deflection zone as a subject breathes. During a particular breathing cycle, the target point may not be within the beam deflection zone long enough to complete a full treatment in half cycle The energy to perform the complete treatment can be distributed in several cycles with required energy $E_1$ to deliver that can be adjusted dynamically based on temperature and/or a thermal dose feedback algorithm. The normalized intensity can be evaluated for each deflection performed to reach each location of the motion trajectory of the target point. The resulting graph will be similar to the one described in Figs. 6 and 7 except that the normalized intensity is display as function of time rather than focal point location as shown in Fig. 11. The relation between time and location is defined by the motion trajectory of the target point.

Fig. 10 shows a planar representation of linear periodic motion 1000. The x-axis is the transducer y-axis displacement 1002. The y-axis is the transducer z-axis displacement 1004. The dashed line 1006 shows the 50% normalized intensity. The dashed line 1008 shows an example of a predetermined threshold 1008. The region 1010 is a beam deflection zone defined by line 1008. The bolded line 1012 shows the sonicated portion of the trajectory 1000.

Fig. 11 illustrates the sonication time for the periodic motion shown in Fig. 10. The normalized intensity is shown as a function of time 1100. The x-axis is the time 1102. The y-axis is the normalized intensity 1104. The dashed line 1006 indicates the 50% level of intensity. The dashed line 1008 indicates the predetermined threshold 1008. When the normalized intensity is above the predetermined threshold 1008 the target is within the beam deflection zone 1010. The period of time 1106 also labeled $T_1$ is the sonication period.

Figs. 10 and 11 illustrate a planar representation of the linear periodic motion trajectory, Fig. 10, or normalized intensity as function of time, Fig. 11, for this trajectory. The thin solid line corresponds to the non sonicated part of the trajectory and the thick solid line corresponds to the sonicated part of the motion trajectory. Short and long dot line corresponds to 50% and $I_1$ normalized intensity threshold respectively. Transducer axis Y corresponds to the FH direction of Figs. 8 and 9.

By performing maximal search, it's possible to find the maximal normalized intensity threshold $I^0_1$ for which all points of the motion trajectory are above this threshold for a duration equal to $T^0_1$ as described in Figs. 10 and 11. The reason why $I^0_1$ and $T^0_1$ are replaced by $I_1$ and $T_1$ on Figs. 10 and 11 is explained further in the text. The threshold $I_1$ has to be preferably selected to a value higher than 50%, otherwise excessive deflection may be required to track the target tissue.

The sonicated part of the tissue motion trajectory, indicated Figs. 10 and 11, will correspond to all points for which the normalized intensity is higher than $I^0_1$ (thick line). Since this part of the motion trajectory corresponds to a duration $T^0_1$, the heating duty cycle $2T^0_1/T_{CYCLE}$ will be sufficient to achieve the require heating. This selection of the part of trajectory heated is thus the one minimizing the intensity drop due to the deflection. The method could also consider more complex cyclic trajectory along 2 or 3 directions. The Figs. 12 and 13 show an example of 2D displacement (which is easier to drawn than 3D displacement). However we can easily understand that the method could be interpolate in 3 directions since the normalized intensity for each point of the motion trajectory can be process in 3D using the same method. Also only half of the motion cycle was considered, assuming that the other half of the cycle is identical, to simplify illustrations. But the complete cycle can be considered if the motion trajectory is not composed of two identical half periods such as trajectories with hysteresis.

Fig. 12 shows a case similar to that shown in Fig. 10. However in this case the planar representation of a curved periodic motion trajectory 1200 is shown. In this example the sonication portion of trajectory 1200 is labeled 1212.

Fig. 13 is analogous to Fig. 11. The normalized intensity is a function of time 1300 of trajectory 1200 is again shown as a function of time. The sonicated period in Fig. 13 is labeled 1306.

On the particular example displayed in Fig. 12 and 13, the motion trajectory of the target point doesn't cross the geometric center of the transducer and thus the normalized intensity never reaches 100% which is not mandatory for this method.

Different strategies could be used to compensate for the intensity drop. The method the most frequently used consists to increase the electrical power applied on the transducer by a factor inversely proportional to the normalized intensity. Thus the intensity at the focal point can be kept constant, for example just below the cavitation threshold.

But if the maximal power is limited by hardware limitation, for example the electrical power apply on the transducer should not be increased, it's possible to increase the sonications duration to a value larger than $T^0_1$.

For such duration increase, it's possible to use an iterative algorithm which takes in to account the average normalized intensity $<I>^0_1$ over the length of the selected part of the motion trajectory. $<I>^0_1$ is a value between $I^0_1$ and 100%.

The algorithm is initiated with value described previously:

$$\begin{cases} T^0_1 = E_1/P_{MAX} \\ I^0_1 \text{ is determined as the threshold above which the normalized intensity } I \text{ remains for a duration} \\ t(I > I^0_1) = T^0_1 \\ (I)^0_1 = \text{average nomalize intensity for this trajectory part} \end{cases}$$

After iteration are performed base on those equations:

$$\begin{cases} T^n_1 = T^0_1/(I)^{n-1}_1 \\ I^n_1 \text{ is determined as the threshold above which the normalized intensity } I \text{ remains for a duration} \\ t(I > I^n_1) = T^n_1 \\ (I)^n_1 = \text{average intensity for this trajectory part} \end{cases}$$

For each iteration, the time $T^n_1$ is increased and thresholds $I^n_1$ and $<I>^n_1$ decreased. Since the time $T^n_1$ can't overpass the half period cycle $T_{CYCLE}/2$, this problem converges. Values obtained at the convergence can be thus labeled $T_1$, $I_1$ and $<I>_1$. The resulting energy deposited at the target is thus $E_1 = P_{MAX} \times <I>_1 \times T_1$.

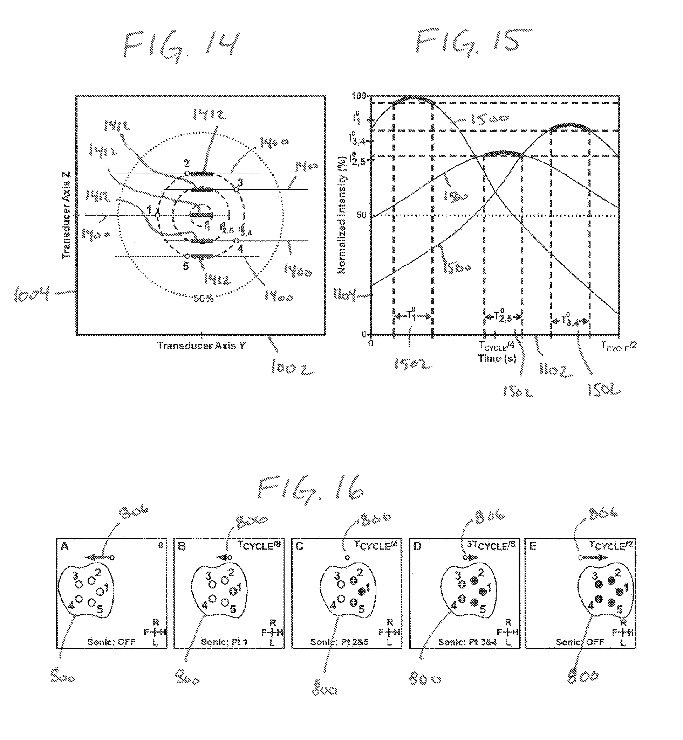

Fig. 14 illustrates a situation similar to that shown in Fig. 10 except in this case there are five points which are sonicated. The trajectories are each labeled 1400 and the sonicated portion of each trajectory is labeled 1412.

Fig. 15 is analogous to Fig. 11. In Fig. 15 three normalized intensity curves 1500 are shown. The portion of the curves labeled 1502 correspond to three sonication periods.

Figs. 14 and 15 show the same representation as Figs. 10 and 11 except that trajectories of 5 points are considered; the position of each point at the middle of the target tissue motion cycle is indicated by small white circles. This corresponds to the location of the points in the tissue. Those 5 points are located along a circular target. The thin solid line corresponds to the non sonicated part of the motion trajectory and the thick solid line corresponds to the sonicated part of the motion trajectory. The target tissue moves from the left to the right along the Y axis during the first half of the cycle.

Fig. 16 is used to illustrate the motion shown in Figs. 14 and 15. Fig. 16 shows a top view of the sonication of five target points moving along the FH direction using motion tracking during part of the displacement. Fig. 16 has frames a, b, c, d and e. Figure 16 shows a top view of sonication of 5 target points moving along FH direction using motion tracking during part of the displacement. The arrow indicates for each time frame the tissue displacement relative to the central position and the point selected for sonications according to the deflection minimization illustrated in Figs. 14 and 15.

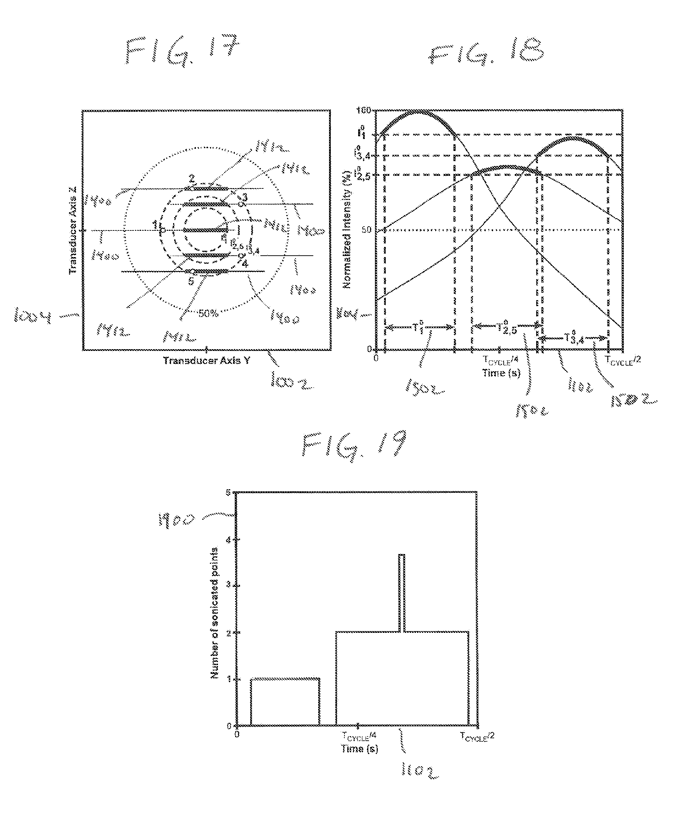

Figs. 17 and 18 show the same representation for example shown in Figs. 14 and 15 except there is higher energy deposition in each point similar maximum intensity criteria. This increases the sonication duty cycle for each point. Figs. 17 and 18 show the same representation as Figs. 14 and 15 with trajectories of 5 points but considering higher energy deposition in each point with similar maximum intensity criteria, thus increasing the sonication duty-cycle for each point.

Volumetric heating:

The method previously described for a single point can be generalized for a target volume including several target points. A temperature and/or dose control algorithm can process the amount of energy $E_K$ to deliver on each point K for a half cycle $T_{CYCLE}/2$ in 2D or in 3D. Using a similar method as previously explained, the minimal required heating duration for each point K could be defined by $T^0_K = E_K/P_{MAX}$. It should be noted that more than one motion cycle is usually necessary to ablate the entire volume (at least for larger volumes) due to hardware restrictions and also in order to avoid causing mechanical damage that may occur at very high ultrasound pressures.

Figs. 17 and 18 illustrates the volumetric motion tracking methods with 5 points located along target circle. To simplify the illustration Fig. 18 with 3 curves instead of 5 curves, it has been assumed that points 2, 5 and 3, 4 are distributes symmetrically with identical amount of energy required and minimum heating duration. A maximal search can be performed, to find the maximal normalized intensity threshold $I^0_K$ for which all points of the motion trajectory of the point K are above this threshold during a duration equal to $T^0_K$:

As illustrated in Fig. 16, it results that at the beginning of the cycle none of the target points is and then only point 1 is sonicated, followed by points 2 and 5 that are sonicated simultaneously, followed by points 3 and 4 that are sonicated simultaneously and at the end of the half cycle no point is sonicated. It results that energy has been delivered in each of the 5 points of the circular sonications trajectory, but deflection has been performed almost only along one axis orthogonal to the motion axis.

To illustrate the fact that this method allows to deliver different amount of energy, Figs. 17 and 18 display a similar exampled of trajectory of 5 points but delivering twice more energy as compare to Figs. 14 and 15 by increasing the sonication duration of each point.

Fig. 19 shows a plot of a number of points which are sonicated as a function of time. The x-axis shows the time 1102 and the y-axis 1900 shows the number of sonicated points as a function of time 1102. Fig. 19 shows the number of points to sonicate during each part time part of the half cycle for the example shown in Figs. 17 and 18. However, in some case more than one point has to be sonicated during the same period such as the points 3 and 4. This can be performed either by forming multiple focal points or switching very fast the focal point from one location to another. The fast switching approach is usually preferred since it induces a lower amount of side lobes than the multiple focus solution (resulting in addition energy loss). In both cases, the total energy deliver by the transducer during this period has to be shared between several points. To take into account this effect a function named $\mathbb{Hb}$, as illustrated Fig. 19, is defined to quantify the number of point to sonicate during each part time part of the cycle.

To compensate for the intensity drop induced by the deflection and the number of point to sonicate in the same period, and iterative algorithm can be used. For duration increase, it's possible to use an iterative algorithm which takes into account the average of the normalized intensity multiply by the number of sonications $<\mathbb{Hb}>_K$ over the length of the selected part of the motion trajectory.

The algorithm is initiated with value described previously:

$$
\begin{cases}
T^0_K = E_K / P_{MAX} \\
I^0_K \text{ is determined as the threshold above which the normalized intensity } I \text{ remains for a duration} \\
\qquad t(I > I^0_K) = T^0_K \\
(\mathbb{Hb})^0_K = \text{average nomalize intensity multiply by number of sonic. for this trajectory part}
\end{cases}
$$

After iteration are performed base on those equations:

$$
\begin{cases}
T^n_K = T^0_K / (\mathbb{Hb})^{n-1}_K \\
I^n_K \text{ is determined as the threshold above which the normalized intensity } I \text{ remains for a duration} \\
\qquad t(I > I^n_K) = T^n_K \\
(\mathbb{Hb})^n_K = \text{average nomalize intensity multiply by number of sonic. for this trajectory part}
\end{cases}
$$

At each iteration, the time $T^n_K$ is increased and threshold $I^n_1$ and $<I>^n_1$ decreased. Since the time $T^n_K$ can't overpass the half period cycle $T_{CYCLE}/2$, this suite converges. Values obtained at the convergence can be thus labeled $T_K$, $I_K$ and $<\mathbb{Hb}>_K$. The resulting energy deposited at the target is thus $E_1 = P_{MAX} \times <\mathbb{Hb}>_1 \times T_1$. As previously thresholds $I_K$ have to be selected preferably to a value higher than 50%, otherway excessive deflection would be required to track the target tissue.

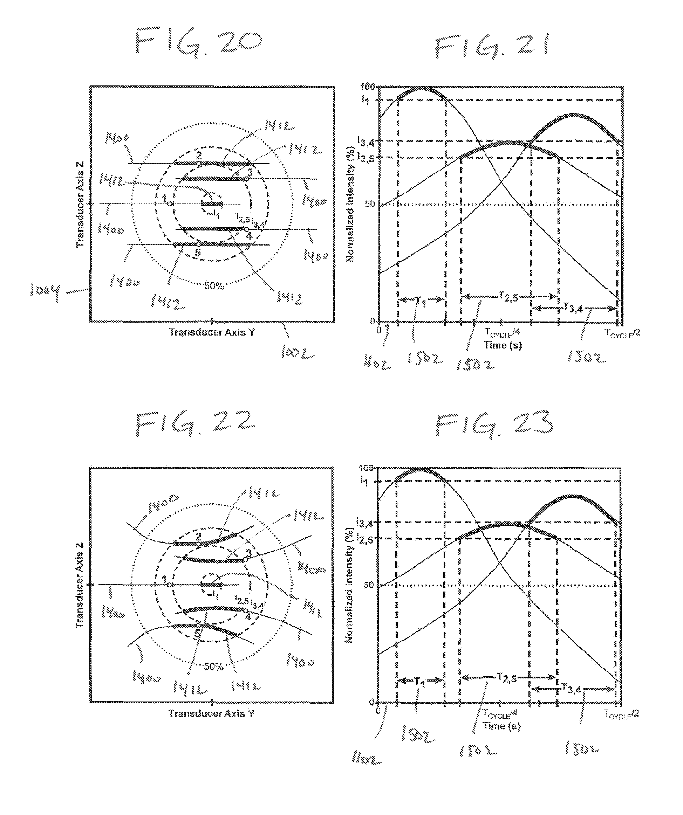

Figs. 20 and 21 are analogous to Figs. 17 and 18. In Figs. 20 and 21 the resulting sonicated trajectories after optimization of the sonication time for the example shown in Figs. 17 and 18 taking into account the number of points sonicated simultaneously. Figs. 20 and 21 shows the resulting sonicated trajectory after optimization of the sonication time for the example shown figure 8 taking in to account the number of point sonicated simultaneously. Relative to figure 8, the duration T1 was reduced and durations T2,5 and T3,4 were increased.

For the example shown in Figs. 17 and 18, it results that the point 1 is never sonicated during the same time period as other points and the associate high average normalized intensity on the selected period would require a much shorter allocated sonicating period than other points as illustrated in Figs. 20 and 21.

It should be noticed that in the examples shown in Figs. 14, 15, 17, 18, 20, and 21 the motion occurs along the axis Y with amplitude larger than the diameter of the target circle. However the deflection performed to deliver the energy on this circle takes place mainly along axis orthogonal to the motion. The deflection along the motion direction is adjusted only to get sufficient energy delivery in each point. It could be that in particular case of volumetric heating is larger than the motion amplitude, in this case additional electronic steering is required along the direction of the motion. Even in such case described algorithm remains exactly the same.

Figs. 22 and 23 are analogous to Figs. 20 and 21. The example illustrated in Figs. 22 and 23 is similar to that in Figs. 20 and 21 except that curve trajectories are used instead of straight ones.

As illustrated in Figs. 22 and 23, this method can be applied using exactly the same algorithm to curved trajectories different for each target point that would be for example the result of elastic displacement of a deformable target organ. For single target point as multiple target point, the required energy $E_K$ to deliver can be adjusted dynamically based on temperature and/or thermal dose feedback algorithm. As consequence the duration $T_K$, the intensity threshold $I_K$ as well as the part of the cycle heated can be also adjusted dynamically (i.e for each cycle).

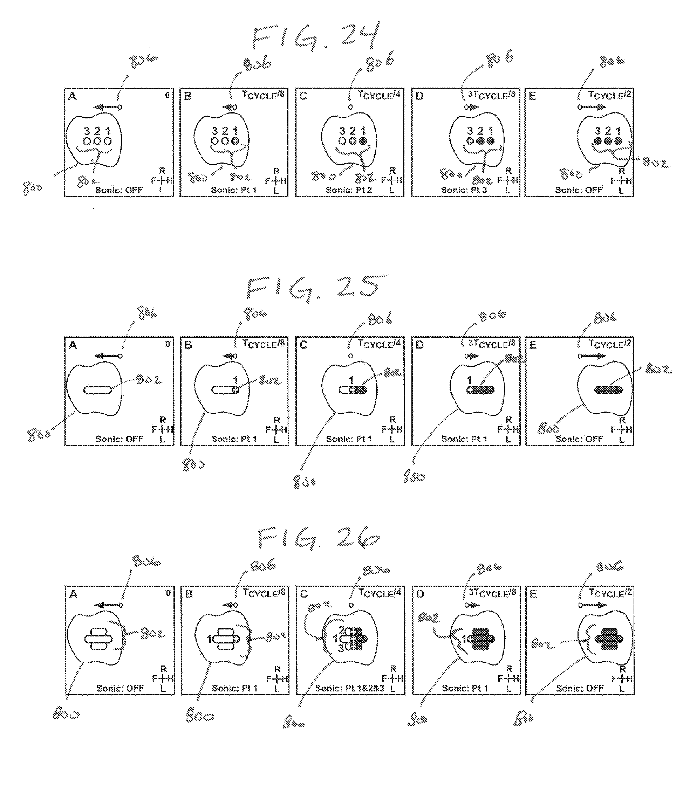

Fig. 24 is similar to Fig. 9 except three targets 802 are being sonicated. Fig. 24 illustrates sonications of 3 target points placed along the motion axis FH while using algorithm to minimize energy loss induced by deflection.

Enlargement of target area along the motion trajectory:

In some case it might be required to perform ablation elongated along the motion trajectory. Such result can be obtained for example by selecting 3 points along the motion trajectory such as the FH axis as displayed in Fig 24. As consequence of algorithm minimizing the deflection previously described, sonications is performed successively in each point when the target points goes in front of the transducer.

Fig. 25 is similar to Fig. 9 except a single point 802 is being sonicated without motion tracking during half of the tissue trajectory to extend the energy delivery to align twice shorter than the amplitude of the motion trajectory. Fig. 25 shows sonication of a single point without motion tracking during half of the tissue trajectory to extend the energy delivery to a line twice shorter than the amplitude of the motion trajectory. Instead of defining several points along the motion trajectory, an alternative algorithm minimizing energy loss induced by deflection could be considered by defining one point only and allowing this sonicated point to spread along the motion trajectory to a control extend. Such algorithm can identify the point of the motion trajectory which the deflection amplitude, and keep sonicating with the same deflection during a part of the motion trajectory in order to cover the target trajectory. As illustrated Fig. 25, sonication is performed always at the same location relatively to the transducer but during only half of the motion-traj ectory in order to deliver energy over a line which is twice shorter than the amplitude of motion. Compared to the first method illustrated Fig. 24, the second methods illustrated Fig. 25 offers the advantage to perform more continuous energy delivery since only one point has to be consider along the motion trajectory rather than a series of points. However the first method allows energy delivery over a sonications trajectory larger than the motion trajectory.

Fig. 26 is similar to Fig. 9 except three points 802 are being sonicated without motion tracking. During a duty cycle adjusted to perform energy delivery over a region over a circular region. Fig. 26 shows the sonication of 3 points without motion tracking with a duty cycle adjusted to perform energy delivery over a region circular region. In the same manner as the first method, the second method can be generalized to several points sonicated. The extension of the energy deliver along the motion direction can be thus controlled individually for each point using different sonications duration for each point. As shown in Fig. 26, the point 1 is used to deliver energy along a longer line than the ones formed by points 2 and 3 in order to deposit energy over a disc. To obtain homogenous energy delivery or energy delivered according to a controller algorithm (for example temperature and/or dose control) the electrical power on the transducer can be modulated as function of deflection amplitude and instantaneous tissue displacement speed. The difference to the examples shown earlier in Figs. 14, 15, 17, 18, 20, and 21 is that there is here no electronic steering of the focal spot along the direction of motion (i.e. the FH direction). Apart from that the sonication methodology is exactly the same.

Cruder algorithm than the one previously described could be considered to minimize the energy loss due to the deflection associated to the motion tracking. For example the user can simply select a normalize intensity threshold $I_T$. Thus the system sonicate the tissue as long as a target point is located within the region where the normalize intensity is higher than the threshold $I_T$. As a consequence the maximum energy deposit is achieved in each point with a deflection limited to the acceptable percentage of energy loss related to deflection. Such maximal energy deliver is useful especially for ablation in highly perfused organ, such liver and kidney, requiring fast temperature rise.

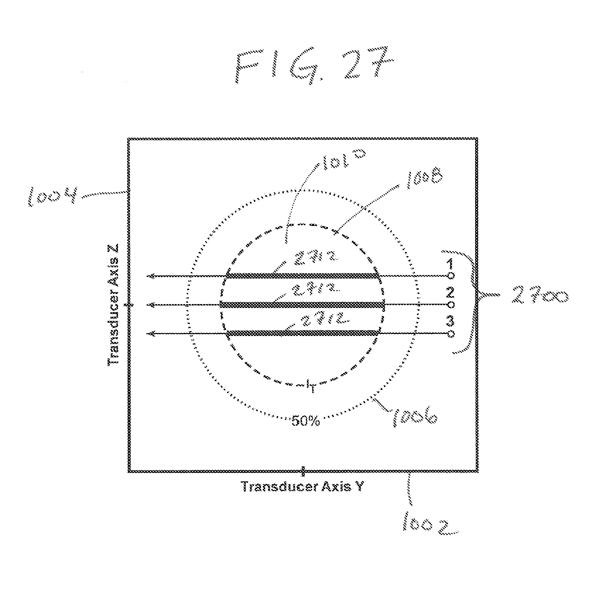

Fig. 27 is analogous to Fig. 10 except in the example shown in Fig. 27 the sonications are for three points with continuous linear displacement based on the normalized intensity threshold 1008. Fig. 27 shows an example of sonications for continuous linear displacement (non periodical) based on normalized intensity threshold region $I_T$. The use of algorithm which minimizes the intensity loss due to deflection can be also applied on organs which are not following a periodic displacement, such as sudden muscle contraction. For example Fig. 27 shows an example of continuous rigid displacement along one axis for 3 points. Motion tacking of the 3 target points is applied unless the intensity threshold is lower than the selected threshold $I_T$.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

LIST OF REFERENCE NUMERALS

| | |
|---|---|
| 300 | therapeutic apparatus |
| 301 | subject |
| 302 | high intensity focused ultrasound system |
| 303 | subject support |
| 304 | fluid filled chamber |
| 306 | ultrasound transducer |
| 308 | mechanism |
| 310 | mechanical actuator/power supply |
| 312 | path of ultrasound |
| 314 | ultrasound window |
| 316 | gel pad |
| 318 | sonication point |
| 320 | moving target |
| 322 | beam deflection zone |
| 324 | computer |
| 326 | hardware interface |
| 328 | processor |

| 330 | user interface |
|---|---|
| 332 | computer storage |
| 334 | computer memory |
| 340 | treatment plan |
| 342 | real time medical data |
| 344 | sonication control commands |
| 346 | ultrasonic calibration |
| 348 | sonication duration |
| 350 | control module |
| 352 | sonication control command generation module |
| 354 | motion tracking module |
| 400 | therapeutic apparatus |
| 402 | magnetic resonance imaging system |
| 404 | magnet |
| 406 | bore of magnet |
| 408 | imaging zone |
| 410 | magnetic field gradient coils |
| 412 | magnetic field gradient coils power supply |
| 414 | radio-frequency coil |
| 416 | transceiver |
| 420 | pulse sequence |
| 422 | magnetic resonance thermometry pulse sequence |
| 424 | magnetic resonance data |
| 426 | magnetic resonance image |
| 428 | temperature map |
| 430 | image reconstruction module |
| 432 | temperature mapping module |
| 434 | image segmentation module |
| 500 | therapeutic apparatus |

| 502 | diagnostic ultrasound system |
| 504 | diagnostic ultrasound transducer |
| 506 | diagnostic ultrasound data |
| 508 | diagnostic ultrasound image |
| 510 | image reconstruction module |
| 512 | image segmentation module |
| 600 | normalized intensity |
| 602 | perpendicular deflection |
| 604 | normalized intensity |
| 606 | 50% normalized intensity |
| 608 | beam deflection zone |
| 700 | normalized intensity |
| 702 | deflection along beam path |
| 704 | beam deflection zone |
| 800 | subj ect |
| 802 | moving target |
| 804 | ultrasound transducer |
| 806 | displacement |
| 808 | ultrasound |
| 1000 | planar representation of linear periodic motion trajectory |
| 1002 | transducer y-axis displacement |
| 1004 | transducer z-axis displacement |
| 1006 | 50% normalized intensity |
| 1008 | predetermined threshold ($I_1$) |
| 1010 | beam deflection zone |
| 1012 | sonicated portion of trajectory |
| 1100 | normalized intensity as a function of time |
| 1102 | time |
| 1104 | normalized intensity |

| 1106 | sonication period |
| 1200 | planar representation of curved period motion trajectory |
| 1212 | sonicated portion of trajectory |
| 1300 | normalized intensity as a function of time |
| 1306 | sonication period |
| 1400 | motion trajectory |
| 1412 | sonicated portion of trajectory |
| 1500 | normalized intensity |
| 1502 | sonication period |
| 2700 | motion trajectory |
| 2712 | sonicated portion of trajectory |

**Claims**

1. A therapeutic apparatus (300, 400, 500) comprising:

- a high intensity focused ultrasound system (302) comprising an ultrasound transducer (306), wherein the ultrasound transducer has an electronically adjustable focus (318), wherein the high intensity focused ultrasound system has a beam deflection zone (322, 608, 704, 1010, wherein the ultrasound transducer is configured for generating acoustic power when supplied with alternating current electrical power, wherein the intensity of ultrasound at the electronically adjustable focus divided by the acoustic power emitted is above a predetermined threshold (606, 1008) within the beam deflection zone;
- a memory (334) for storing machine executable instructions (350, 352, 354); and
- a processor (328) configured for controlling the therapeutic apparatus, wherein execution of the machine executable instructions causes the processor to:

- receive (102, 202) real time medical data (342, 424, 506), wherein the real time medical data is descriptive of the location of a moving target (320, 802);
- adjust (104, 204) the electronically adjustable focus to target the moving target using the real time medical data; and
- sonicate (106, 206) the moving target when the moving target is within the beam deflection zone.

2. The therapeutic apparatus of claim 1, wherein the moving target is located at least partially using real time tracking of the target within the real time medical data.

3. The therapeutic apparatus of claim 1 or 2, wherein execution of the instructions causes the processor to receive a motion tracking model (354), wherein the motion tracking model is configured for predicting a location of the moving target using the real time medical data, wherein the electronically adjustable focus is adjusted at least partially in accordance with the location predicted by the motion tracking model.

4. The therapeutic apparatus of claim 1, 2, or 3, wherein the moving target comprises at least one localized volume.

5. The therapeutic apparatus of claim 4, wherein the moving target comprises multiple localized volumes, wherein the electronically adjustable focus is targeted to the moving target by specifying a sequence of localized volumes chosen from the multiple localized volumes, wherein execution of the instructions further cause the processor to determine the sequence is in accordance with the real time medical data.

6. The therapeutic apparatus of claim 4 or 5, wherein execution of the instructions further causes the processor to receive an ultrasonic calibration (346), wherein the ultrasonic calibration is descriptive of the spatially dependent intensity of ultrasound at the electronically adjustable focus within the beam deflection zone, wherein execution of the instructions further causes the processor to determine a sonication duration (348, 1106, 1306, 1502) for each localized volume in accordance with the ultrasonic calibration and the real time medical data, wherein the therapeutic apparatus is configured to sonicate each localized volume for the sonication duration.

7. The therapeutic apparatus of claim 1, 2, or 3, wherein the moving target comprises at least one path.

8. The therapeutic apparatus of claim 7, wherein the electronically adjustable focus follows a trajectory along the at least one path, wherein execution of the instructions further cause the processor to determine the trajectory is in accordance with the real time medical data.

9. The therapeutic apparatus of claim 7 or 8, wherein execution of the instructions further causes the processor to receive an ultrasonic calibration (346), wherein the ultrasonic calibration is descriptive of the spatially dependent intensity of ultrasound at the electronically adjustable focus within the beam deflection zone, wherein each of the at least one paths is divided into portions, wherein execution of the instructions further causes the processor to determine a sonication (348, 1106, 1306, 1502) duration in accordance with the ultrasonic calibration and the real time medical data for each of the portions, wherein the therapeutic apparatus is configured to sonicate each of the portions for the sonication duration.

10. The therapeutic apparatus of any one of the preceding claims, wherein the location of the moving target is predicted using dynamic analysis of the real time medical imaging data.

11. The therapeutic apparatus of any one of the preceding claims, wherein the real time medical data is real time medical image data, wherein execution of the instructions further causes the processor to acquire (202) the real time medical image data using a medical imaging system (402, 502), wherein the medical imaging system is any one of the following: a magnetic resonance imaging system (402) and a diagnostic ultrasound imaging system (502).

12. The therapeutic apparatus of claim 11, wherein the medical image data comprises motion data and real time thermographic data (428), wherein the moving target is located using the motion data.

13. The therapeutic apparatus of claim 12, wherein the sonication of the moving target is controlled in accordance with the thermographic data.

14. A method of operating a therapeutic apparatus (300, 400, 500), wherein the therapeutic apparatus comprises a high intensity focused ultrasound system (302) comprising an ultrasound transducer (306), wherein the ultrasound transducer has an electronically adjustable focus (318), wherein the high intensity focused ultrasound system has a beam deflection zone (322, 608, 704, 1010), wherein the ultrasound transducer is configured for generating acoustic power when supplied with alternating current electrical power, wherein the intensity of ultrasound at the electronically adjustable focus divided by the acoustic power emitted is above a predetermined threshold (606, 1008) within the beam deflection zone, wherein the method comprises the steps of:

- receiving (102, 202) real time medical data (342, 352, 354), wherein the real time medical data is descriptive of the location of a moving target (320, 802);
- adjusting (104, 204) the electronically adjustable focus to target the moving target using the real time medical data; and
- sonicating (106, 206) the moving target when the moving target is within the beam deflection zone.

15. A computer program product comprising machine executable instructions (350, 352, 354) for operating a therapeutic apparatus (300, 400, 500), wherein the therapeutic apparatus comprises a high intensity focused ultrasound system (302) comprising an ultrasound transducer (306), wherein the ultrasound transducer has an electronically adjustable focus (318), wherein the high intensity focused ultrasound system has a beam deflection zone (322, 608, 704, 1010), wherein the ultrasound transducer is configured for generating acoustic power when supplied with alternating current electrical power, wherein the intensity of ultrasound at the electronically adjustable focus divided by the acoustic power emitted is above a predetermined threshold (606, 1008) within the beam deflection zone, wherein the therapeutic apparatus further comprises a processor configured for controlling the therapeutic apparatus, wherein execution of the machine executable instructions causes the processor to:

- receive (102, 202) real time medical data (342, 424, 506), wherein the real time medical data is descriptive of the location of a moving target (320, 802);
- adjust (104, 204) the electronically adjustable focus to target the moving target using the real time medical data; and
- sonicate (106, 206) the moving target when the moving target is within the beam deflection zone.

FIG. 1

```
        ┌─────────────┐
        │    start    │ ~100
        └──────┬──────┘
               │
               ▼
┌──────────────────────────────────────────┐
│        receive real time medical data     │ ~102
└──────────────────┬───────────────────────┘
                   │
                   ▼
┌──────────────────────────────────────────┐
│ adjust the electronically adjustable focus to target │ ~104
│ the moving target using the real time medical data   │
└──────────────────┬───────────────────────┘
                   │
                   ▼
┌──────────────────────────────────────────┐
│ sonicate the target when the moving target is within │ ~106
│           the beam deflection zone                   │
└──────────────────┬───────────────────────┘
                   │
                   ▼
              ~108
No      ╱ sonication ╲   Yes      ┌────────┐
    ◄───  ╲ finished? ╱   ───────►│  end   │
           ╲        ╱              └────────┘
                                        ~110
```

## FIG. 2

```
              ┌─────────────┐
              │    start    │── 200
              └──────┬──────┘
                     │
  ┌──────────────────▼──────────────────────────┐
  │  acquire real time medical data using a      │── 202
─►│           medical imaging system             │
│ └──────────────────┬──────────────────────────┘
│                    │
│ ┌──────────────────▼──────────────────────────┐
│ │  adjust the electronically adjustable focus  │── 204
│ │  to target the moving target using the real  │
│ │              time medical data               │
│ └──────────────────┬──────────────────────────┘
│                    │
│ ┌──────────────────▼──────────────────────────┐
│ │  sonicate the target when the moving target  │── 206
│ │      is within the beam deflection zone      │
│ └──────────────────┬──────────────────────────┘
│                    │
│              ┌──────▼─────┐ 208
│         No  ╱ sonication   ╲  Yes     ┌─────────┐
└───────────◄   finished?     ►───────► │   end   │── 210
            ╲               ╱           └─────────┘
             ╲─────────────╱
```

FIG.3

# FIG. 4

FIG. 5

FIG. 6

FIG. 7

604

606

600

608

Normalized Intensity (%)

Deflection perpendicular to the beam: axis Y or Z

602

604

606

700

704

Normalized Intensity (%)

Deflection along the beam: axis X

702

EP 2 574 375 A1

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

| A | 0 | B | TCYCLE/8 | C | TCYCLE/4 | D | 3TCYCLE/8 | E | TCYCLE/2 |
|---|---|---|---|---|---|---|---|---|---|

Sonic: OFF | Sonic: Pt 1 | Sonic: Pt 2 | Sonic: Pt 3 | Sonic: OFF

FIG. 25

| A | 0 | B | TCYCLE/8 | C | TCYCLE/4 | D | 3TCYCLE/8 | E | TCYCLE/2 |
|---|---|---|---|---|---|---|---|---|---|

Sonic: OFF | Sonic: Pt 1 | Sonic: Pt 1 | Sonic: Pt 1 | Sonic: OFF

FIG. 26

| A | 0 | B | TCYCLE/8 | C | TCYCLE/4 | D | 3TCYCLE/8 | E | TCYCLE/2 |
|---|---|---|---|---|---|---|---|---|---|

Sonic: OFF | Sonic: Pt 1 | Sonic: Pt 1&2&3 | Sonic: Pt 1 | Sonic: OFF

FIG. 27

**Europäisches Patentamt**
**European Patent Office**
**Office européen des brevets**

# EUROPEAN SEARCH REPORT

Application Number

EP 11 18 2849

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 97/22015 A1 (PHILIPS ELECTRONICS NV [NL]; PHILIPS NORDEN AB [SE]) 19 June 1997 (1997-06-19) | 1,2,4,5, 7,8, 10-13,15 | INV. A61N7/02 A61B19/00 |
| Y | * the whole document * | 6,9 | |
| X | US 2009/112132 A1 (CHANG HSU [TW] ET AL) 30 April 2009 (2009-04-30) * paragraphs [0005], [0007], [0020], [0021], [0044], [0048] * * figures 1,6 * | 1,3-5,7, 8,15 | |
| Y | US 2010/222676 A1 (OGIHARA MAKOTO [JP] ET AL) 2 September 2010 (2010-09-02) * abstract * * paragraphs [0033], [0035] * | 6,9 | |

TECHNICAL FIELDS SEARCHED (IPC)

A61N
A61B

The present search report has been drawn up for all claims

| Place of search | Date of completion of the search | Examiner |
|---|---|---|
| Munich | 5 March 2012 | Willig, Hendrik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 11 18 2849

This annex lists the patent family members relating to the patent documents cited in the above-mentioned European search report.
The members are as contained in the European Patent Office EDP file on
The European Patent Office is in no way liable for these particulars which are merely given for the purpose of information.

05-03-2012

| Patent document cited in search report | | Publication date | Patent family member(s) | | Publication date |
|---|---|---|---|---|---|
| WO 9722015 | A1 | 19-06-1997 | DE | 69634976 D1 | 01-09-2005 |
| | | | DE | 69634976 T2 | 20-04-2006 |
| | | | EP | 0811171 A1 | 10-12-1997 |
| | | | JP | 3902233 B2 | 04-04-2007 |
| | | | JP | 4188395 B2 | 26-11-2008 |
| | | | JP | H11500948 A | 26-01-1999 |
| | | | JP | 2007203033 A | 16-08-2007 |
| | | | US | 5938600 A | 17-08-1999 |
| | | | WO | 9722015 A1 | 19-06-1997 |
| US 2009112132 | A1 | 30-04-2009 | NONE | | |
| US 2010222676 | A1 | 02-09-2010 | US | 2010222676 A1 | 02-09-2010 |
| | | | WO | 2006137484 A1 | 28-12-2006 |

EPO FORM P0459

For more details about this annex : see Official Journal of the European Patent Office, No. 12/82